# EUROPEAN PATENT APPLICATION

(11) **EP 4 814 097 A1**
(43) Date of publication of application: **30.09.2026**
(21) Application number: 26167004.6
(22) Date of filing: 24.03.2026
(51) Int. Cl.: C12M 1/26

(54) **ASPIRATION SYSTEM, PROGRAM, AND ASPIRATION METHOD**

(30) Priority: 25.03.2025 JP 2025050623
(71) Applicant: Yokogawa Electric Corporation, Tokyo 180-8750 (JP)
(72) Inventor: NISHIDE, Kohki, Musashino-shi, Tokyo 180-8750 (JP)
(74) Representative: Zimmermann, Tankred Klaus

(57) **Abstract**

An aspiration system (1) for aspirating internal components of a cell from a sample (62) in a container includes a processor (11) that punctures a target position of the cell a plurality of times with a puncture tool, then after puncturing the target position of the cell a plurality of times, controls an aspiration unit (32) having a tip (33) for aspiration attached thereto to aspirate internal components of the cell with the tip (33), and stores the internal components aspirated by the tip (33) in a storage (351).

## Description

### CROSS-REFERENCE TO RELATED APPLICATION

The present application claims priority to Japanese Patent Application No. 2025-050623 filed on March 25, 2025, the entire contents of which are incorporated herein by reference.

### TECHNICAL FIELD

The present disclosure relates to an aspiration system, a program, and an aspiration method.

### BACKGROUND

Automatic cell aspiration devices that capture images of cells seeded in a container such as a well plate (microplate) and automatically aspirate any one or more cells, cell clumps, parts of cells, or tissue slices are known. Patent Literature (PTL) 1 describes a support system that supports the task of aspirating specific cells or cell components from a group of cells in a cell culture container.

### CITATION LIST

### Patent Literature

PTL 1: JP 2023-017601 A

### SUMMARY

When the cell membrane or internal matrix of the cell from which the sample is to be collected is strong, it may be difficult to aspirate the internal components from the cell, making it difficult to collect a sufficient amount of the internal components. Conventional configurations thus have room for improvement in terms of harvesting a sufficient amount of the internal components of a cell when the cell membrane or matrix is strong.

An aim of the present disclosure is to enable the harvesting of a sufficient yield of the internal components of a cell even when the cell membrane or matrix of the cell is strong.

An aspiration system according to several embodiments is
(1) an aspiration system for aspirating internal components of a cell from a sample in a container, the aspiration system comprising:
   a processor configured to
      puncture a target position of the cell a plurality of times with a puncture tool;
      after puncturing the target position of the cell a plurality of times, control an aspiration unit having a tip for aspiration attached thereto to aspirate internal components of the cell with the tip; and
      store the internal components aspirated by the tip in a storage.
   In one embodiment,
(2) in the aspiration system according to (1),
   the processor may control a position of the aspiration unit to puncture the target position of the cell a plurality of times with the tip as the puncture tool.
   In one embodiment,
(3) in the aspiration system according to (1) or (2),
   the processor may
      accept a set number of punctures from a user; and
      after puncturing the target position of the cell the set number of punctures with the puncture tool, aspirate the internal components of the cell with the tip.
   In one embodiment,
(4) in the aspiration system according to (3),
   in a case in which the number of punctures is not set by the user, the processor may aspirate the internal components of the cell with the tip after puncturing the target position of the cell a preset number of times with the puncture tool.
   In one embodiment,
(5) in the aspiration system according to (1) or (2),
   the processor may determine a number of times to puncture the target position of the cell according to a type of the sample set by a user.
   In one embodiment,
(6) in the aspiration system according to (1) or (2),
   the processor may
      acquire a captured image of the sample captured by an imager before puncturing with the puncture tool; and
      analyze the captured image to determine a number of times to puncture the target position of the cell.
   In one embodiment,
(7) in the aspiration system according to (1) or (2),
   the processor may
      acquire a captured image of the sample captured by an imager each time the cell is punctured by the puncture tool; and
      puncture the target position of the cell with the puncture tool until the captured image of the sample satisfies a predetermined condition.
   In one embodiment,
(8) in the aspiration system according to any one of (1) to (7),
   the processor may
      acquire a yield of the internal components stored in the storage; and
      aspirate the internal components of the cell with the tip again in a case in which a cumulative value of the acquired yield of the internal components is smaller than a predetermined threshold.
   In one embodiment,
(9) in the aspiration system according to any one of (1) to (8),
   the processor may puncture the target position of the cell a plurality of times by repeating an operation of lowering the puncture tool to a predetermined position of the cell and an operation of raising the puncture tool a certain distance.
   In one embodiment,
(10) in the aspiration system according to any one of (1) to (9),
   the processor may puncture a plurality of different positions in a horizontal direction centered on the target position of the cell with the puncture tool.
   A program according to several embodiments is
(11) a program for controlling operation of an aspiration system for aspirating internal components of a cell from a sample in a container, the aspiration system comprising a processor,
   the program being configured to cause the processor to execute operations comprising:
   puncturing a target position of the cell a plurality of times with a puncture tool;
   controlling, after puncturing the target position of the cell a plurality of times, an aspiration unit having a tip for aspiration attached thereto to aspirate internal components of the cell with the tip; and
   storing the internal components aspirated by the tip in a storage.
   In one embodiment,
(12) in the program according to (11),
   the processor may
      accept a set number of punctures from a user; and
      aspirate the internal components of the cell with the tip after puncturing the target position of the cell the set number of punctures with the puncture tool.
   In one embodiment,
(13) in the program according to (12),
   in a case in which the number of punctures is not set by the user, the processor may aspirate the internal components of the cell with the tip after puncturing the target position of the cell a preset number of times with the puncture tool.
   In one embodiment,
(14) in the program according to (11),
   the processor may determine a number of times to puncture the target position of the cell according to a type of the sample set by a user.
   In one embodiment,
(15) in the program according to (11),
   the processor may
      acquire a captured image of the sample captured by an imager before puncturing with the puncture tool; and
      analyze the captured image to determine a number of times to puncture the target position of the cell.
   In one embodiment,
(16) in the program according to (11),
   the processor may
      acquire a captured image of the sample captured by an imager each time the cell is punctured by the puncture tool; and
      puncture the target position of the cell with the puncture tool until the captured image of the sample satisfies a predetermined condition.
   In one embodiment,
(17) in the program according to any one of (11) to (16),
   the processor may
      acquire a yield of the internal components stored in the storage; and
      aspirate the internal components of the cell with the tip again in a case in which a cumulative value of the acquired yield of the internal components is smaller than a predetermined threshold.
   In one embodiment,
(18) in the program according to any one of (11) to (17),
   the processor may puncture the target position of the cell a plurality of times by repeating an operation of lowering the puncture tool to a predetermined position of the cell and an operation of raising the puncture tool a certain distance.
   In one embodiment,
(19) in the program according to any one of (11) to (18),
   the processor may puncture a plurality of different positions in a horizontal direction centered on the target position of the cell with the puncture tool.
   An aspiration method according to several embodiments is
(20) an aspiration method, for aspirating internal components of a cell from a sample in a container, to be performed by an aspiration system, the aspiration method comprising:
   puncturing a target position of the cell a plurality of times with a puncture tool;
   controlling, after puncturing the target position of the cell a plurality of times, an aspiration unit having a tip for aspiration attached thereto to aspirate internal components of the cell with the tip; and
   storing the internal components aspirated by the tip in a storage.

According to one embodiment of the present disclosure, a sufficient yield of the internal components of a cell can be harvested even when the cell membrane or matrix of the cell is strong.

### BRIEF DESCRIPTION OF THE DRAWINGS

In the accompanying drawings:
FIG. 1 is a schematic diagram illustrating a configuration example of an aspiration system according to an embodiment;
FIG. 2 is a schematic diagram illustrating an example of the operation of the aspiration system according to an embodiment;
FIG. 3 is a schematic diagram illustrating an example of the operation of the aspiration system according to an embodiment;
FIG. 4 is a schematic diagram illustrating an example of the operation of the aspiration system according to an embodiment;
FIG. 5 is a schematic diagram illustrating an example of the operation of the aspiration system according to an embodiment; and
FIG. 6 is a flowchart illustrating an example of the operation of the aspiration system according to an embodiment.

### DETAILED DESCRIPTION

### <Embodiment>

An embodiment of the present disclosure will be described below, with reference to the drawings. In each drawing, parts having the same configuration or function are labeled with the same reference numerals. In the description of the present embodiment, repetitive descriptions of the same parts may be omitted or simplified as appropriate.

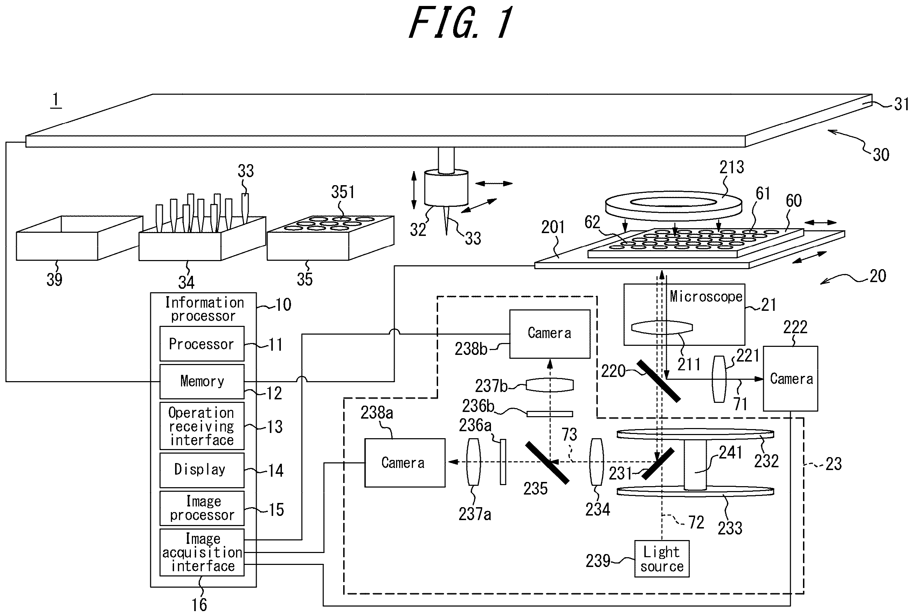

FIG. 1 is a schematic diagram illustrating a configuration example of an aspiration system 1 according to an embodiment. The aspiration system 1 is a device that supports the process of aspirating cellular components of cells, intracellular organelles, cell slices, and the like (hereinafter collectively referred to as "cells and the like"). The aspiration system 1 includes an information processor 10, an optical system 20, and an aspiration operation unit 30.

The optical system 20 includes an XY stage 201, a microscope 21, a bright-field illumination source 213, a dichroic mirror (DM) 220, a variable magnification lens 221, a camera 222 as an imager, and a confocal scanner 23. The XY stage 201 has a cell culture container placed thereon. In the present embodiment, the cell culture container placed on the XY stage 201 is, for example, a well plate 60 having a plurality of wells (holes) 61 formed therein, but it may alternatively be a cell culture dish, a cover glass chamber, a petri dish, or the like. A specimen (sample) 62 such as a cell is placed in the wells 61 of the well plate 60. The microscope 21 is provided with an objective lens 211.

The bright-field illumination source 213, the DM 220, the variable magnification lens 221, and the camera 222 constitute a bright-field optical system for performing bright-field observation. In bright-field observation, bright-field signal light 71 is irradiated from the bright-field illumination source 213 toward the well plate 60. The DM 220 is designed to reflect the bright-field signal light 71 and transmit an excitation light beam 72 and fluorescent signal 73, described later. The bright-field signal light 71 passes through the microscope 21, is reflected by the DM 220, and is imaged by the camera 222 for bright-field observation using the variable magnification lens 221. Based on an exposure signal from the information processor 10, the camera 222 exposes the image projected onto the light receiving surface of a two-dimensional sensor thereof for a specified period of time and converts the image into digital image data. An image acquisition interface 16 of the information processor 10 acquires digital image data from the camera 222. The bright-field illumination source 213 has a space formed in the center and has, for example, an annular (donut) shape.

The confocal scanner 23 has dichroic mirrors (DM) 231, 235, a pinhole array disk (Nipkow disk) 232, a microlens array disk (ML disk) 233, relay lens 234, bandpass filters 236 (236a, 236b), lenses 237 (237a, 237b), cameras 238 (238a, 238b) as an imager, and a light source 239. In fluorescent observation, an excitation light source 239 emits an excitation light beam 72 having a specific wavelength toward the well plate 60. A fluorescent reagent is added to the sample 62, and when the sample 62 is irradiated with the excitation light beam 72, a fluorescent signal 73 having a longer wavelength than the excitation light beam 72 is emitted from the excited sample 62.

The DM 231 is designed to transmit the excitation light beam 72 and reflect a desired fluorescent signal 73. On the ML disk 233, a plurality of light-collecting optical elements (microlenses) are arranged, for example, in a spiral shape. The Nipkow disk 232 has a plurality of pinholes disposed at positions where the excitation light beam 72 is focused by the plurality of light-collecting optical elements of the ML disk 233. Based on a control signal transmitted from the information processor 10, the confocal scanner 23 can use a motor or the like to rotate the Nipkow disk 232 and the ML disk 233 at high speed about the central axis of rotation while the disks are mechanically connected to each other by a member 241. Here, the individual microlenses and pinholes are arranged so that the individual pinholes formed on the Nipkow disk 232 sweep across the surface of the sample 62. Therefore, the Nipkow disk 232 functions as a scanning unit that scans the illumination light (excitation light beam 72) over the sample 62. The Nipkow disk 232 rotates the plurality of pinholes in a plane substantially perpendicular to the optical axis of the excitation light beam 72. During the imaging operation, the ML disk 233 and the Nipkow disk 232 are constantly rotating.

Most of the excitation light beam 72 that has passed through the ML disk 233 passes through the Nipkow disk 232. Therefore, in the confocal scanner 23, by using the ML disk 233 and the Nipkow disk 232 in combination, the irradiation intensity of the excitation light beam 72 on the sample 62 is increased compared to when the Nipkow disk 232 is used alone. Furthermore, reflection of the excitation light beam 72 at portions of the Nipkow disk 232 other than the pinhole is suppressed. Therefore, the signal-to-noise ratio of the image of the sample 62 is increased.

The excitation light beam 72 is focused into individual light beams by the ML disk 233, passes through the DM 231, and then passes through the individual pinholes of the Nipkow disk 232. The excitation light beam 72 passes through the DM 220 and is focused by the objective lens 211 of the microscope 21 onto the sample 62 placed in the well 61 of the well plate 60. As described above, each of the samples 62 in the well plate 60 has a fluorescent reagent added thereto. The fluorescent signal 73 emitted by each fluorescent reagent in the sample 62 again passes through the objective lens 211 and the DM 220 and is focused on individual pinholes in the Nipkow disk 232.

The fluorescent signal 73 that passes through the pinhole in the Nipkow disk 232 forms a confocal image. The fluorescent signal 73 is reflected by the DM 231, passes through the relay lens 234 and the lenses 237 (237a, 237b), and forms an image on the camera 238 (238a, 238b) for fluorescent observation.

In the example of FIG. 1, in order to support simultaneous use of the light source 239 for excitation of a plurality of wavelengths, the confocal scanner 23 includes a DM 235 having the characteristic of dispersing the fluorescent signal 73. Furthermore, the confocal scanner 23 includes the bandpass filters 236 (236a, 236b) for improving the S/N ratio of the image and for passing only a necessary wavelength band of the fluorescent signal 73. The wavelengths of the fluorescent signal 73 emitted by the sample 62 vary, and it is desirable to prepare a plurality of bandpass filters 236 corresponding to the required wavelengths using, for example, filter foil or the like.

The plane on which the pinholes of the Nipkow disk 232 are arranged, the surface of the sample 62, and the light receiving surface of the two-dimensional sensor of the cameras 238 (238a, 238b) are arranged in an optically conjugate relationship with each other. Therefore, an optical cross-sectional image of the sample 62, that is, a confocal image, is formed on the two-dimensional sensor of the cameras 238. Based on an exposure signal from the information processor 10, the cameras 238 expose the image projected onto the light receiving surface of a two-dimensional sensor thereof for a specified period of time and convert the image into digital image data. The image acquisition interface 16 of the information processor 10 acquires image data from the cameras 238.

In the present embodiment, the optical system 20 has a configuration for performing confocal two-color fluorescent observation and bright-field observation, for example, but the configuration of the optical system 20 is not limited to this. For example, the optical system 20 may have a configuration of one color of epi-fluorescence without including the confocal scanner 23 and the bright-field observation system, a configuration of only one color of confocal light, or a configuration of one color of confocal light and a bright field. Furthermore, the optical system 20 may be configured to acquire an image of the sample 62 using an observation method other than the fluorescent observation, bright-field observation, and epi-fluorescent observation.

The aspiration operation unit 30 includes an XYZ stage 31, an aspiration unit 32, a tip rack 34, a sample rack 35, and a disposal box 39. A plurality of tips 33 for aspirating cells or the like are arranged on the tip rack 34. The sample rack 35 has a storage 351 to store the tips 33 that have aspirated cells or the like, or to store the cells or the like aspirated using the tips 33. The disposal box 39 is a box for discarding the tips 33 to be discarded.

The aspiration unit 32 is moved in the X-axis direction, the Y-axis direction, and the Z-axis direction by the XYZ stage 31. The X-axis direction and the Y-axis direction are two directions perpendicular to each other on a plane perpendicular to the optical axis of the excitation light beam 72. The Z-axis direction is parallel to the optical axis of the excitation light beam 72. When performing the operation of aspirating a cell or the like, the aspirating unit 32 obtains and attaches a tip 33 from the tip rack 34. The aspiration unit 32 then moves in the direction in which the well plate 60 is located and aspirates a sample 62 such as a cell from a well 61 at a predetermined aspiration location. The aspiration location can be, for example, on the optical axis of the microscope 21.

At this time, the XY stage 201 moves the well plate 60 so that the sample 62 to be aspirated is positioned at the aspiration location. As a result, the tip 33 and the sample 62 to be aspirated are vertically overlapped. The bright-field illumination source 213 is annular so as not to interfere with the aspiration unit 32 during the aspiration operation. After aspirating the cell, the aspirating unit 32 moves toward the sample rack 35 and stores, in the storage 351 of the sample rack 35, the cell or the like aspirated into the tip 33 or the tip 33 that has aspirated the cell or the like.

The description now returns to FIG. 1. The information processor 10 controls the overall operation of the aspiration system 1. The information processor 10 can be configured using an information processing device such as a PC (Personal Computer). The information processor 10 includes a processor 11, a memory 12, an operation receiving interface 13, a display 14, an image processor 15, and the image acquisition interface 16.

The processor 11 includes one or more processors. In the present embodiment, the "processor" can be a general-purpose processor or a dedicated processor specialized for particular processing, but the processor is not limited to these examples. The processor 11 is communicably connected to each component of the information processor 10 and controls the operation of the information processor 10 as a whole. Furthermore, the processor 11 controls each component of the information processor 10, thereby controlling the overall operation of the aspiration system 1. For example, the processor 11 controls the operations of the microscope 21, the bright-field illumination source 213, the camera 222, the confocal scanner 23, the XY stage 201, the XYZ stage 31, and the like.

The memory 12 includes any storage module, such as a hard disk drive (HDD), a solid state drive (SSD), a read-only memory (ROM), a random access memory (RAM), and the like. The memory 12 may function as, for example, a main storage device, an auxiliary storage device, and a cache memory. The memory 12 stores any information used in the operation of the information processor 10 or obtained as a result of the operation of the information processor 10. The memory 12 records, for example, the image data acquired by the image acquisition interface 16 and the analysis results of the image processor 15.

The operation receiving interface 13 receives various operations from the user. The operation receiving interface 13 is realized by, for example, a physical key, a capacitance key, a pointing device, a touch screen provided integrally with the display of the display 14, or a microphone that receives voice input.

The display 14 performs display processing of the images acquired by the image acquisition interface 16, the analysis results of the image processor 15, and the like. The display 14 is realized by, for example, a liquid crystal display, an organic EL (Electro-Luminescence) display, or the like.

The image processor 15 performs image processing on the image data acquired by the image acquisition interface 16 and then performs various analyses. Specifically, the image processor 15 recognizes cells and cell organelles by template matching or the like, and calculates features such as size, brightness, protein amount, and ion amount for each identified cell. Furthermore, the image processor 15 uses the calculated features to perform processing such as listing and graphing information about cells and the like.

The image acquisition interface 16 acquires image data from the cameras 222, 238 (238a, 238b).

The functions of the information processor 10 can be realized by having a processor included in the processor 11 execute a program (computer program) that can be used to cause the aspiration system 1 according to the present embodiment to function. That is, the functions of the information processor 10 can be realized by software. The program causes the computer to execute the processing in the steps included in the operations of the information processor 10, thereby causing the computer to realize the functions corresponding to the processing in each step. That is, the program is a program for causing a computer to function as the information processor 10 according to the present embodiment.

The program can be recorded in a computer-readable recording medium. The computer-readable recording medium is, for example, a magnetic recording device, an optical disk, a magneto-optical recording medium, or a semiconductor memory. The program can be distributed, for example, by selling, transferring, or lending a portable recording medium, such as a DVD (Digital Versatile Disc) or a CD-ROM (Compact Disc ROM), on which the program is recorded. The program may be distributed by storing the program in a server storage and transferring the program from the server to other computers via a network. The program may be provided as a program product.

The computer temporarily stores a program recorded on a portable recording medium, or a program transferred from a server, for example, in a main storage device. The computer then uses a processor to read the program stored in the main storage device and execute processing in accordance with the read program. The computer may read the program directly from the portable recording medium and execute processing in accordance with the program. The computer may execute processing in accordance with the received program each time the program is transferred from the server to the computer. Such processing may be performed by a so-called ASP (Application Service Provider) type service, which realizes functions simply by issuing execution commands and obtaining results, without transferring a program from the server to the computer. The program encompasses information used for processing by a computer and equivalents to a program. For example, data that is not a direct instruction for a computer but has the nature of specifying computer processing falls under the category of "equivalents to a program".

A part or all of the functions of the information processor 10 may be realized by a dedicated circuit included in the processor 11. That is, some or all of the functions of the information processor 10 may be realized by hardware. Moreover, the information processor 10 may be realized by a single information processing device or may be realized by a plurality of information processing devices working in cooperation with each other.

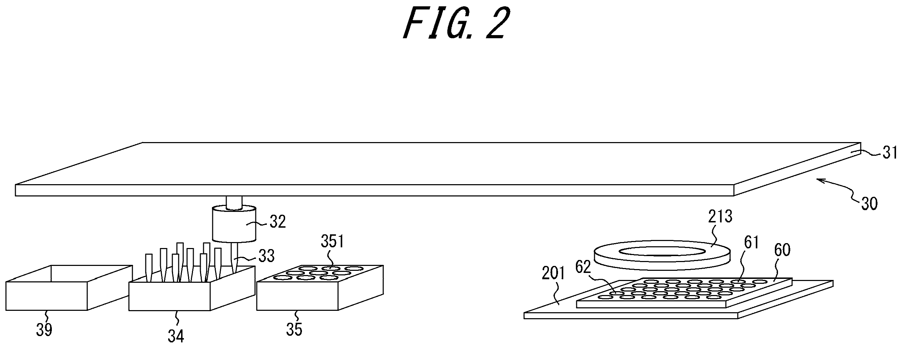

Next, the operation of the aspiration system 1 will be outlined with reference to FIGS. 2 to 5. FIGS. 2 to 5 are schematic diagrams illustrating an example of the operation of the aspiration system 1 according to one embodiment. First, the aspiration system 1 receives the setting of the well plate 60 on which the sample 62 is placed. At least one cell is located in the well 61 of the well plate 60. The aspiration system 1 determines which cells have specific attributes from among these cells, aspirates the determined cells, and discharges them onto a sample rack 35. Specifically, each well 61 on the well plate 60 is imaged, and the captured image is analyzed to detect a cell or the like that is a candidate to be aspirated. The aspiration system 1 displays an image of the detected cell or the like on the display 14 and receives, from the user, a selection of the target cell or the like to be aspirated. At this time, the aspiration system 1 also accepts a selection of whether to turn on a pooling function for storing the aspirated cell or the like in the same storage 351 of the sample rack 35. When aspirating a cell or the like, the aspiration system 1 also captures another image of the cell or the like to be aspirated and accepts a selection as to whether to perform position correction to correct the position of aspiration based on the recaptured image.

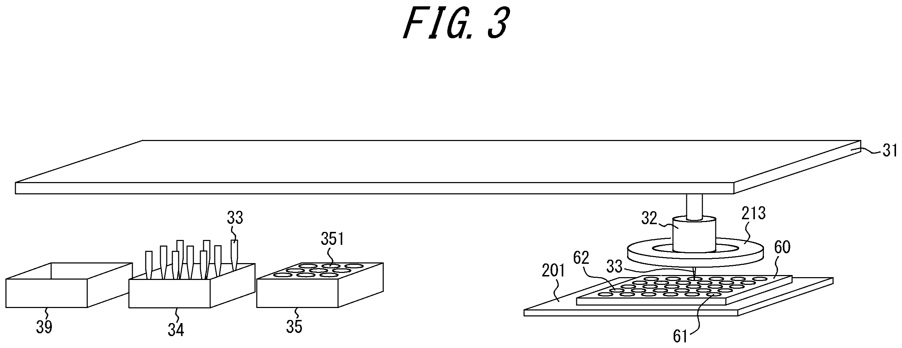

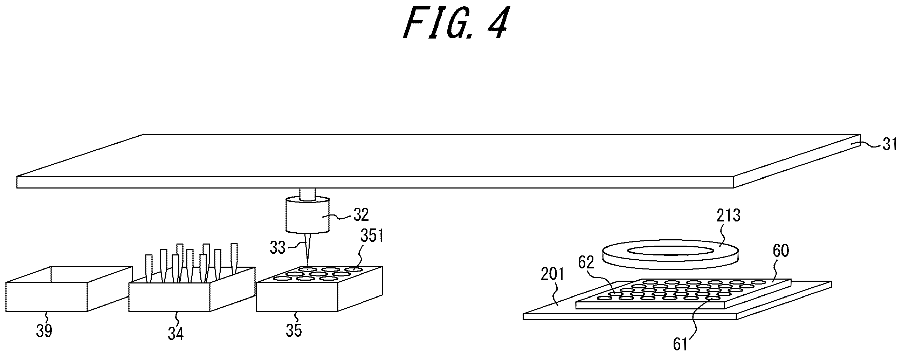

The aspiration system 1 starts an operation of aspirating cells or the like in response to a user operation on the operation receiving interface 13 of the information processor 10. The aspiration system 1 moves the aspiration unit 32 to above the tip rack 34 using the XYZ stage 31 and attaches the tip 33 to the aspiration unit 32 (FIG. 2). Next, the aspiration system 1 moves the aspiration unit 32 to above the well plate 60 using the XYZ stage 31. The aspiration system 1 moves the well plate 60 using the XY stage 201 so that the target cell or the like is positioned under the tip 33 and then aspirates the cell or the like (FIG. 3). After aspirating the cell or the like, the aspiration system 1 moves the aspiration unit 32 to above the sample rack 35 using the XYZ stage 31. When the pooling function is set to ON, the aspiration system 1 can discharge the aspirated cell or the like to a specific storage 351 to which a plurality of cells or the like is discharged (FIG. 4). After discharging the cell or the like, the aspiration system 1 moves the aspiration unit 32 to above the disposal box 39 using the XYZ stage 31 and releases the tip 33 to discard the tip 33. The aspiration system 1 executes such a series of operations, i.e., mounting the tip 33, aspirating the cell or the like, discharging the cell or the like, and discarding the tip 33, for each of the cells or the like to be aspirated.

In this manner, the aspiration system 1 according to the present disclosure operates as an automatic cell aspiration device capable of switching the pooling function ON/OFF. The pooling function is a function that enables a plurality of cells or the like having the same attribute to be discharged into the same storage 351 of the sample rack 35. The aspiration system 1 may, for example, display a check box on the display 14 of the information processor 10 and be able to switch the pooling function ON/OFF depending on the user's selection of the check box via the operation receiving interface 13. Alternatively, the aspiration system 1 may be configured to set a storage 351a on the sample rack 35 for each well 61 of the well plate 60 in which the cell or the like to be aspirated is placed. When the same storage 351a is set for a plurality of cells or the like, the aspiration system 1 may control storage in accordance with the setting. This allows the aspiration system 1 to store a plurality of cells or the like having the same attribute in the same storage 351a or the like.

The pooling function may be used in applications such as PCR and cloning. When performing such an analysis, the aspiration system 1 discharges and stores the number of cells or the like required for the analysis into the same storage 351 of the sample rack 35 such as a well plate. The user removes the stored cells or the like from the aspiration system 1 for analysis or storage in an incubator (culture device). In order to ensure a sufficient number of cells for such applications and enable analysis with sufficient accuracy, the aspiration system 1 may be capable of setting the number of cells or the like to be pooled in the same storage 351a to any value. For example, when accepting the selection of the target cell or the like to be aspirated, the aspiration system 1 may also accept the selection of a storage destination and generate a list including information such as the target cell or the like and the storage destination. For example, when the sample rack 35 is a well plate having 8 × 12 (i.e., 96) wells arranged in a grid pattern, the wells serving as the storage 351 of the sample rack 35 can be identified by two-dimensional coordinates (X, Y). Here, X may take any value from A to H, and Y may take any value from 1 to 12, for example. Information on the storage destination of cells or the like may, for example, be recorded in the list as information such as (A, 1). For example, if two targets are registered in the list and the storage destination for each is set to (A, 1), then the two cells or the like will be pooled in the well (A, 1).

In addition to the operation mode compatible with the pooling function as described above, the aspiration system 1 may, according to user selection, also switch to and execute an operation mode not compatible with the pooling function. In the operation mode compatible with the pooling function, the aspiration system 1 stores cells and the like aspirated by the tip 33 in the sample rack 35, but in the operation mode not compatible with the pooling function, the tip 33 that aspirated the cell or the like is itself stored in the sample rack 35. That is, the operation mode compatible with the pooling function is an operation mode for preserving the aspirated cells and the like, and the operation mode not compatible with the pooling function is an operation mode for preserving the tip 33 into which the cell or the like has been aspirated. In the operation mode not compatible with the pooling function, the aspiration system 1 does not discharge the cell or the like from the tip 33 but rather places the tip 33 into which the cell or the like has been aspirated in the storage 351 of the sample rack 35. For example, in applications such as direct MS (Mass Spectrometry), the operation mode not compatible with the pooling function is selected, and the tip 33 into which the cell or the like has been aspirated is used for analysis as is. Even in the operation mode compatible with the pooling function, the aspiration system 1 may store the aspirated cells or the like in different storages 351 without using the pooling function.

The aspiration system 1 may perform position correction when aspirating a sample 62 such as a cell from the well plate 60. This makes it possible to address the movement and division of cells, which occur over time between the capturing of an image for selecting a target and the aspiration of the cell or the like, and enables accurate sampling. The aspiration system 1 performs position correction based on image analysis in the image processor 15, but instead, position correction may be performed by aspirating the cell or the like at a position specified by the user on the operation receiving interface 13. The aspiration system 1 may be capable of specifying position corrections on the order of 0.1 µm to several hundred µm.

After aspirating the cell or the like, the aspiration system 1 may select whether to store the cell or the like in the sample rack 35 based on image analysis or a user instruction. If the user determines based on the state in which cell or the like has been aspirated that the sample does not need to be stored, the user can simply discard the sample, thereby avoiding contamination of the stored sample. For example, when sampling and pooling intracellular material, if an entire cell is sampled, pooling that cell as is will result in extra material being mixed in with what was pooled until just before. Therefore, whether to store such aspirated material can be determined by the user by looking at the image immediately after sampling or can be determined by image processing. The aspiration system 1 may perform such a determination for each aspirated cell or the like or may perform the determination only for specific cells or the like. For example, the aspiration system 1 may display a check box on the display 14 to confirm whether to store the cell or the like that is aspirated and may determine whether to store the cell or the like that is aspirated depending on whether the check box is selected by the user. When storage of cells or the like is selected, the aspiration system 1 stores the cells or the like in the storage 351, which is at a pre-specified position in the sample rack 35.

The aspiration system 1 may count the number of cells or the like stored (the number of samples) for each storage 351 of the sample rack 35. For example, the aspiration system 1 may manage the number of samples of cells or the like stored in each storage 351 and may increment the number of samples in a storage 351 by one each time a cell or the like is stored in that storage 351. The aspiration system 1 may display the number of samples in each storage 351 on the display 14 in response to a user instruction. This allows the user to easily check the number of samples stored in each storage 351.

As described above, the aspiration system 1 aspirates internal components of a cell from a sample 62 in a container such as a well plate 60. The sample 62 may be one or more cells (animal/plant) or tissues (such as slices of organs). Here, the aspiration system 1 punctures a target position of the cell a plurality of times with a puncture device, and after puncturing the target position of the cell a plurality of times, controls the aspiration unit 32 having the tip 33 for aspiration attached thereto to aspirate the internal components of the cell with the tip 33. The aspiration system 1 stores the internal components aspirated by the tip 33 in the storage 351. The puncture tool may be the tip 33 for aspiration or any other member such as a needle.

In this way, the aspiration system 1 punctures the target position of the cell a plurality of times to deform or weaken the cell membrane and internal matrix of the cell before performing the aspiration operation, thereby increasing the yield of internal components by the aspiration operation. Therefore, the aspiration system 1 can collect a sufficient amount of internal components of cells even when the membrane or matrix of the cells is strong.

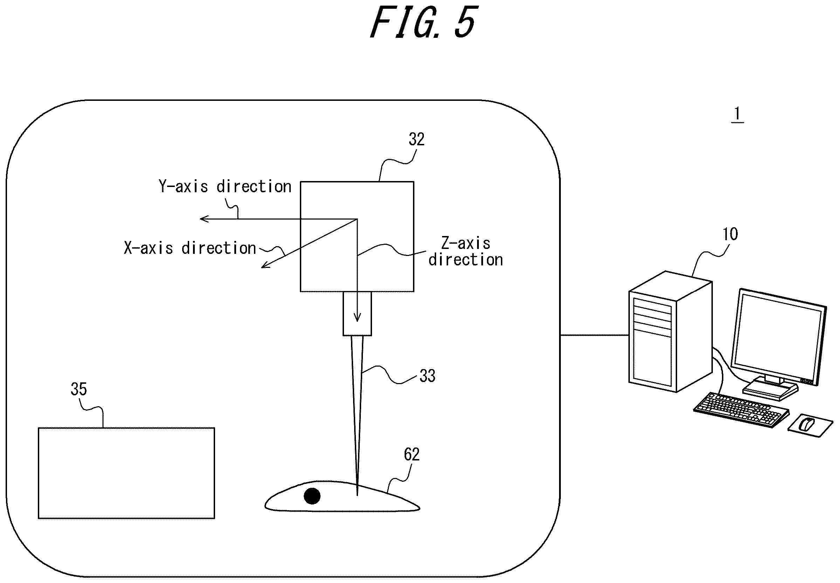

As illustrated in FIG. 5, the aspiration system 1 moves the aspiration unit 32 under the control of the processor 11 of the information processor 10 to aspirate internal components of a cell from the sample 62. The aspiration system 1 may move the aspiration unit 32 up and down in the Z-axis direction to puncture the target position of the cell a plurality of times. Alternatively, the aspiration system 1 may shift the aspiration unit 32 in at least one of the X-axis direction and the Y-axis direction each time puncturing is performed. After aspirating the internal components of the cells from the sample 62, the aspiration system 1 discharges the aspirated material into the storage 351 of the sample rack 35 for storage.

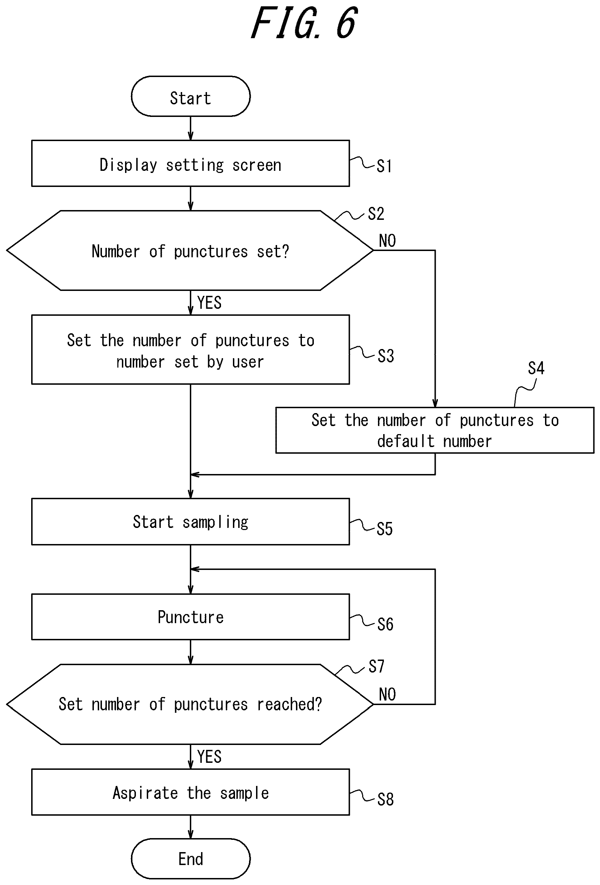

FIG. 6 is a flowchart illustrating an example of the operation of the aspiration system 1 according to an embodiment. The operation of the aspiration system 1, described with reference to FIG. 6, corresponds to one of the support methods according to the present embodiment. The operation of each step in FIG. 6 is executed under the control of the processor 11 of the information processor 10. A program for causing a computer to execute the support method according to the present embodiment may include the steps illustrated in FIG. 6.

In step S1, the processor 11 displays a setting screen on the display 14 and receives settings related to the aspiration of the sample 62 from the user. The settings on the setting screen may include the number of times the target position of the cell is punctured.

In step S2, the processor 11 determines whether the number of punctures has been set by the user. In a case in which the number of punctures has been set (YES in step S2), the processor 11 proceeds to step S3. Otherwise (NO in step S2), the processor 11 proceeds to step S4.

In step S3, the processor 11 sets the number of punctures to the number set by the user.

In step S4, the processor 11 sets the number of punctures to a default number. The default number is preset in the memory 12.

In step S5, the processor 11 starts sampling the sample 62 according to the procedure described above with reference to FIGS. 2 to 5. Sampling may be a sequence of processes including puncturing and aspirating the target, and storing or discharging the aspirated material.

In step S6, the processor 11 punctures the cell of the sample 62. That is, the processor 11 moves the puncture tool (including the tip 33) to the target position of the cell and punctures the target position of the cell.

In step S7, the processor 11 determines whether the cell of the sample 62 has been punctured the number of times set in step S3 or step S4. In a case in which the set number of punctures has been reached (YES in step S7), the processor 11 proceeds to step S8. In a case in which the set number of punctures has not been reached (NO in step S7), the processor 11 returns to step S6 and punctures again. Specifically, the processor 11 may raise the aspiration unit 32 a certain distance (for example, about 50 µm) in the Z-axis direction to move the puncture tool away from the target, and then lower the aspiration unit 32 again to puncture the target again. Alternatively, the processor 11 may perform puncturing by shifting the aspiration unit 32 in the X-axis and Y-axis directions.

In step S8, the processor 11 aspirates the internal components of the cell of the sample 62 using the tip 33. In a case in which the cell in the sample 62 is punctured with a puncture tool other than the tip 33, the processor 11 replaces the puncture tool with the tip 33 and performs the cell aspirating operation. The processor 11 may discharge the aspirated internal components into the storage 351 of the sample rack 35 or the like for storage. When the process of step S8 is completed, the processor 11 ends the process of the flowchart.

As described above, the aspiration system 1 aspirates internal components of a cell from a sample 62 in a container such as a well plate 60. Here, the aspiration system 1 punctures the target position of the cell a plurality of times with the puncture tool. After puncturing the target position of the cell a plurality of times, the aspiration system 1 controls the aspiration unit 32 having the tip 33 for aspiration attached thereto to aspirate internal components of the cell with the tip 33. The aspiration system 1 stores the internal components aspirated by the tip 33 in the storage 351.

In this way, the aspiration system 1 punctures the target position of the cell a plurality of times to deform or weaken the cell membrane and internal matrix of the cell before performing the aspiration operation, thereby increasing the yield of internal components by the aspiration operation. Therefore, the aspiration system 1 can collect a sufficient amount of internal components of cells even when the membrane or matrix of the cells is strong.

The aspiration system 1 may control the position of the aspiration unit 32 to puncture the target position of the cell a plurality of times with the tip 33 as the puncture tool. With this configuration, the internal components of the cell can be aspirated directly using the tip 33 without replacing the puncture tool with the tip 33, and the desired sample 62 can be collected in a short time.

The aspiration system 1 may accept a set number of punctures from the user, and after puncturing the target position of the cell the set number of punctures with the puncture tool, may aspirate the internal components of the cell with the tip 33. With this configuration, the user can set the number of punctures and therefore can select an appropriate number of punctures according to the type of sample 62.

In a case in which the number of punctures is not set by the user, the aspiration system 1 may aspirate the internal components of the cell with the tip 33 after puncturing the target position of the cell a preset number of times with the puncture tool. With this configuration, in a case in which the number of punctures is not set by the user, the aspiration system 1 can puncture the number of times set as a default and aspirate the internal components of the cell.

The aspiration system 1 may also determine the number of times to puncture the target position of the cell according to the type of sample 62 set by the user. For example, the memory 12 may store a correspondence relationship between the type of sample 62 and the number of punctures in advance. When the type of sample 62 is selected by the user on a setting screen or the like displayed in step S1, the aspiration system 1 may set the number of punctures corresponding to that type of sample 62 as the number of punctures to be performed during sampling. With this configuration, the aspiration system 1 can determine an appropriate number of punctures according to the type of sample 62 set by the user and perform the puncturing.

The aspiration system 1 may also acquire a captured image of the sample 62 captured by an imager (such as the camera 222, 238) before puncturing with the puncture tool. The aspiration system 1 may analyze the captured image to determine the number of times to puncture the target position of the cell. For example, the memory 12 may store a trained model that has been trained in advance to receive input of a captured image of the sample 62 and to output the number of punctures required to aspirate the internal components of a cell from the sample 62 depicted in the captured image. The aspiration system 1 may input the captured image of the sample 62 into such a trained model to determine the number of punctures. With this configuration, the aspiration system 1 can determine the appropriate number of punctures based on the captured image of the unpunctured sample 62 and then perform the puncturing.

The aspiration system 1 may acquire a captured image of the sample 62 captured by an imager each time the cell is punctured by the puncture tool. The aspiration system 1 may puncture the target position of the cell with the puncture tool until the captured image of the sample 62 satisfies a predetermined condition. For example, the memory 12 may store a trained model that has been trained in advance to receive input of a captured image of the sample 62 and to output whether deformation of the cell membrane or matrix of the sample 62 depicted in the captured image can be confirmed. The aspiration system 1 may input a captured image of the sample 62 into such a trained model and determine whether the captured image of the sample 62 satisfies a predetermined condition. With this configuration, the aspiration system 1 refers to the captured image of the sample 62 each time puncturing is performed and continues puncturing until deformation or the like of the cell membrane or matrix can be confirmed, thereby allowing puncturing to be performed an appropriate number of times.

The aspiration system 1 may also acquire the yield of the internal components stored in the storage 351. The yield of the internal components may be measured by a sensor such as an imager or a weight sensor. When the cumulative value of the yield of the obtained internal components is smaller than a predetermined threshold, the aspiration system 1 may again cause the tip 33 to aspirate the internal components of a cell. With this configuration, the aspiration system 1 can collect the internal components until a required yield is reached.

The aspiration system 1 may also puncture the target position of the cell a plurality of times by repeating an operation of lowering the puncture tool to a predetermined position of the cell and an operation of raising the tip of the puncture tool a certain distance. With this configuration, the aspiration system 1 repeatedly punctures the target position of the cell by raising and lowering the puncture tool, thereby effectively deforming or weakening the membrane and internal matrix of the cell.

The aspiration system 1 may control the position of the aspiration unit 32 to puncture a plurality of different positions in the horizontal direction centered on the target position of the cell with the puncture tool. With this configuration, the aspiration system 1 can puncture a plurality of different positions in the horizontal direction near the target position, effectively deforming or weakening the membrane and internal matrix of the cell.

As described above, the aspiration system 1 deforms or weakens the membrane and internal matrix of a cell by puncturing the target of the sample 62 a plurality of times, thereby increasing the yield of the sample by aspiration. As a result, samples necessary for analyses such as mass spectrometry and PCR can be collected in a short time, and the overall experiment time including these analyses can be shortened. By increasing the yield from the same cell, more accurate analysis can be performed than when samples are collected from a plurality of cells and analyzed. Also, since the puncture settings and control are managed using software, even in the case of using an existing aspiration system 1, the operation of the aspiration system 1 according to the present embodiment can be achieved simply by updating the program.

The present disclosure is not limited to the embodiments described above. For example, a plurality of blocks described in the block diagrams may be integrated, or a block may be divided. Instead of executing a plurality of steps described in the flowcharts in chronological order in accordance with the description, the plurality of steps may be executed in parallel or in a different order according to the processing capability of the apparatus that executes each step, or as required. Other modifications can be made without departing from the spirit of the present disclosure.

Also, for example, the configuration and operation of the information processor 10 may be distributed among a plurality of computers capable of communicating with each other.

## Claims

1. An aspiration system for aspirating internal components of a cell from a sample in a container, the aspiration system comprising:
a processor configured to
puncture a target position of the cell a plurality of times with a puncture tool;
after puncturing the target position of the cell a plurality of times, control an aspiration unit having a tip for aspiration attached thereto to aspirate internal components of the cell with the tip; and
store the internal components aspirated by the tip in a storage.

2. The aspiration system according to claim 1, wherein the processor controls a position of the aspiration unit to puncture the target position of the cell a plurality of times with the tip as the puncture tool.

3. The aspiration system according to claim 1 or 2, wherein the processor
accepts a set number of punctures from a user; and
aspirates the internal components of the cell with the tip after puncturing the target position of the cell the set number of punctures with the puncture tool.

4. The aspiration system according to claim 3, wherein in a case in which the number of punctures is not set by the user, the processor aspirates the internal components of the cell with the tip after puncturing the target position of the cell a preset number of times with the puncture tool.

5. The aspiration system according to claim 1 or 2, wherein the processor determines a number of times to puncture the target position of the cell according to a type of the sample set by a user.

6. The aspiration system according to claim 1 or 2, wherein the processor
acquires a captured image of the sample captured by an imager before puncturing with the puncture tool; and
analyzes the captured image to determine a number of times to puncture the target position of the cell.

7. The aspiration system according to claim 1 or 2, wherein the processor
acquires a captured image of the sample captured by an imager each time the cell is punctured by the puncture tool; and
punctures the target position of the cell with the puncture tool until the captured image of the sample satisfies a predetermined condition.

8. The aspiration system according to any one of claims 1 to 7, wherein the processor
acquires a yield of the internal components stored in the storage; and
aspirates the internal components of the cell with the tip again in a case in which a cumulative value of the acquired yield of the internal components is smaller than a predetermined threshold.

9. The aspiration system according to any one of claims 1 to 8, wherein the processor punctures the target position of the cell a plurality of times by repeating an operation of lowering the puncture tool to a predetermined position of the cell and an operation of raising the puncture tool a certain distance.

10. The aspiration system according to any one of claims 1 to 9, wherein the processor punctures a plurality of different positions in a horizontal direction centered on the target position of the cell with the puncture tool.

11. A program for controlling operation of an aspiration system for aspirating internal components of a cell from a sample in a container, the aspiration system comprising a processor,
the program being configured to cause the processor to execute operations comprising:
puncturing a target position of the cell a plurality of times with a puncture tool;
controlling, after puncturing the target position of the cell a plurality of times, an aspiration unit having a tip for aspiration attached thereto to aspirate internal components of the cell with the tip; and
storing the internal components aspirated by the tip in a storage.

12. The program according to claim 11, wherein the processor
accepts a set number of punctures from a user; and
aspirates the internal components of the cell with the tip after puncturing the target position of the cell the set number of punctures with the puncture tool.

13. The program according to claim 12, wherein in a case in which the number of punctures is not set by the user, the processor aspirates the internal components of the cell with the tip after puncturing the target position of the cell a preset number of times with the puncture tool.

14. The program according to claim 11, wherein the processor determines a number of times to puncture the target position of the cell according to a type of the sample set by a user.

15. An aspiration method, for aspirating internal components of a cell from a sample in a container, to be performed by an aspiration system, the aspiration method comprising:
puncturing a target position of the cell a plurality of times with a puncture tool;
controlling, after puncturing the target position of the cell a plurality of times, an aspiration unit having a tip for aspiration attached thereto to aspirate internal components of the cell with the tip; and
storing the internal components aspirated by the tip in a storage.
